# EUROPEAN PATENT SPECIFICATION

(11) **EP 2 504 477 B1**
(45) Date of publication and mention of the grant of the patent: **06.06.2018**
(21) Application number: 10784401.1
(22) Date of filing: 22.11.2010
(51) Int. Cl.: D04H 13/00, A61L 27/50, B29C 61/06

(54) **ARTICLES AND METHODS USING SHAPE-MEMORY POLYMERS**
ARTIKEL UND VERFAHREN UNTER VERWENDUNG FORMGEDÄCHTNISPOLYMERE
ARTICLES ET PROCÉDÉS UTILISANT DES POLYMÈRES À MÉMOIRE DE FORME

(30) Priority: 24.11.2009 US 264024 P
(43) Date of publication of application: 03.10.2012
(73) Proprietor: 3M Innovative Properties Company, St. Paul, MN 55133-3427 (US)
(72) Inventor: RULE, Joseph, D., Saint Paul, Minnesota 55133-3427 (US); LEWANDOWSKI, Kevin M., Saint Paul, Minnesota 55133-3427 (US); SEBASTIAN, John M., Saint Paul, Minnesota 55133-3427 (US); MARTIN, Philip G., Saint Paul,, Minnesota 55133-3427 (US); ANGADJIVAND, Seyed A., Saint Paul, Minnesota 55133-3427 (US); JONES, Marvin E., Saint Paul, Minnesota 55133-3427 (US)
(74) Representative: Herzog, Fiesser & Partner Patentanwälte PartG mbB
(86) International application number: PCT/US2010/057632
(87) International publication number: WO 2011/066224

(56) References cited:
- EP-A1- 1 528 133
- EP-A2- 0 367 039
- GB-A- 2 470 185
- JP-A- 2009 225 930
- US-A1- 2002 115 977

## Description

### BACKGROUND

When a shape-memory material is initially formed it adopts an intrinsic shape. If the shape-memory material is then heated at or above a transition temperature (Tₜᵣₐₙₛ) it softens and will deform in response to an applied external stress. If the shape-memory material is cooled in this state, it will retain its strained temporary shape indefinitely. If the strained shape-memory material is reheated to a sufficiently high temperature, higher than the transition temperature, the strained shape-memory material returns to its intrinsic shape.

Some polymeric materials are shape-memory materials. For convenience, such polymeric shape-memory materials will be referred to hereinafter as shape-memory polymers (SMPs). The secret behind SMPs lies in their molecular network structure, which typically contains physical or chemical crosslinks.

In some cases, the physical crosslinks are formed by at least two separate phases. One phase with the highest thermal transition, Tᵤₚₚₑᵣ, determines the temperature that must be exceeded to reestablish physical crosslinks responsible for the intrinsic shape. A second phase includes switching segments with the ability to soften above a certain transition temperature (Tₜᵣₐₙₛ) and is responsible for the temporary shape. In some cases, Tₜᵣₐₙₛ is near the glass transition temperature (T_{g}), and in other cases it is near the melting temperature (Tₘ) of the SMP. Exceeding Tₜᵣₐₙₛ (while remaining below Tᵤₚₚₑᵣ) softens the switching segments, allowing the SMP to resume its intrinsic shape. Below Tₜᵣₐₙₛ, flexibility of the segments is at least partly limited.

In other cases, the polymer is chemically crosslinked. These chemical crosslinks are often covalent bonds. These chemical crosslinks can be formed as the polymer is initially cured, often by including a multifunctional monomer in the polymerization mixture. Alternatively, the chemical crosslinks can be formed after the initial polymerization, for example by radiation such as UV light or E-beam. The intrinsic shape of chemically crosslinked shape memory polymers is fixed as the crosslinks are formed, and this intrinsic shape of these chemically crosslinked polymers can usually not be changed even at extreme temperatures.

### SUMMARY

The invention is defined in the appended claims. In one aspect, the present disclosure provides an article comprising: a nonwoven fiber web; and a strip bonded to the nonwoven fiber web, wherein the strip comprises a shape-memory polymer, wherein the strip has a strained temporary shape and an intrinsic shape, and wherein if heated at or above a transition temperature, the strip at least partially converts from the strained temporary shape to the intrinsic shape, thereby causing at least one of folding, cupping, or puckering of the nonwoven fiber web.

In another aspect, the present disclosure provides a method of making an article comprising bonding a strip to a nonwoven fiber web, wherein the strip comprises a shape-memory polymer, wherein the strip has a strained temporary shape and an intrinsic shape, and wherein if heated at or above a transition temperature, the strip at least partially converts from the strained temporary shape to the respective intrinsic shape, thereby causing at least one of folding or puckering of the nonwoven fiber web.

The strip may be bonded to the nonwoven fiber web at discrete points along the strip. The strip may be bonded to the nonwoven fiber web continuously along the strip. The strip and the nonwoven fiber web may be flat. The strip may be curved.

The present invention provides an article comprising: a nonwoven fiber web; and strips bonded to the nonwoven fiber web, wherein the strips comprise a shape-memory polymer, wherein each strip respectively has a strained temporary shape and an intrinsic shape, and wherein if heated at or above a transition temperature, the strips at least partially convert from their respective strained temporary shape to their respective intrinsic shape, thereby causing at least one of folding or puckering of the nonwoven fiber web, wherein at least a portion of the strips are interconnected.

In another aspect, the present invention provides a method of making an article comprising bonding strips to a nonwoven fiber web, wherein the strips comprise a shape-memory polymer, wherein each strip respectively has a strained temporary shape and an intrinsic shape, and wherein if heated at or above a transition temperature, the strips at least partially convert from their respective strained temporary shape to their respective intrinsic shape, thereby causing at least one of folding, cupping, or puckering of the nonwoven fiber web, wherein at least some of the strips are interconnected.

In some embodiments, the strips have respective lengths, and wherein at least one of the strips is bonded to the nonwoven fiber web at discrete points. In some embodiments, the strips have respective lengths, and wherein at least one of the strips is continuously bonded to the nonwoven fiber web. In some embodiments, the strips and the nonwoven fiber web are flat. In some embodiments, the strips are linear. In some embodiments, the strips are disposed entirely on one major surface of the nonwoven fiber web. In some embodiments, the strips are disposed on opposed major surfaces of the nonwoven fiber web. In some embodiments, at least some of the strips are disposed in a parallel arrangement. In some embodiments, the strips comprise a mesh.

In some embodiments, methods according to the present disclosure further comprise heating the strip or strips at or above the transition temperature.

In another aspect, the present disclosure provides an article prepared according to a method of the present disclosure. In some embodiments, the article comprises a respirator mask or a pleated filter.

Advantageously, articles and methods according to the present disclosure are useful for preparing various three-dimensional nonwoven articles, however in their present form they are substantially flat, and can be efficiently packaged, stored, and transported accordingly; for example, as stacked sheets or as a roll.

As used herein:
the term "bonded" means securely affixed to, for example, by an adhesive or mechanical fastener, welding, etc. and does not necessarily imply direct contact, although direct contact is permissible;
the term "cupping" means forming one or more cup shapes;
the term "pucker" means to become gathered or contracted and wrinkled; and
the term "strip" refers to an elongated segment which may comprise straight and/or curved portions, which may or may not have uniform width and/or thickness, and which may be independent of, or interconnected with (as in the case of a mesh), other strips.

The features and advantages of the present disclosure will be understood upon consideration of the detailed description as well as the appended claims. These and other features and advantages of the disclosure may be described below in connection with various illustrative embodiments of the disclosure. The above summary is not intended to describe each disclosed embodiment or every implementation of the present disclosure. The Figures and the detailed description which follow more particularly exemplify illustrative embodiments.

### BRIEF DESCRIPTION OF THE DRAWINGS

Fig. 1 is a process flow diagram showing a method of making an exemplary article according to the present disclosure.
Figs. 2A - 2C are, respectively, a schematic top view, a schematic bottom view, and a schematic side view of an exemplary article according to the present disclosure prior to heating to its transition temperature.
Figs. 3A - 3C are, respectively, a schematic top view, a schematic bottom view, and a schematic side view of an exemplary article shown in Figs. 2A-2C after heating to its transition temperature.
Figs. 4A - 4C are, respectively, a schematic top view, a schematic bottom view, and a schematic side view of an exemplary article according to one embodiment of the present disclosure prior to heating to its transition temperature.
Figs. 5A - 5C are, respectively, a schematic top view, a schematic bottom view, and a schematic side view of an exemplary article according to the embodiment shown in Figs. 4A-4C after heating to its transition temperature.
Fig. 6 is a process flow diagram showing a method of making an exemplary article according to the present disclosure.
Fig. 7A - 7B are, respectively, a cross-sectional back view and a cross-sectional side view of an exemplary article according to one embodiment of the present disclosure before heating to its transition temperature.
Fig. 8A - 8B are, respectively, a cut-away back view and a cross-sectional side view of an exemplary article according to the embodiment shown in Figs. 7A-7B after heating to its transition temperature.
Fig. 9A - 9B are, respectively, a back view and a cross-sectional side view of an exemplary article according to one embodiment of the present disclosure before heating to its transition temperature.
Fig. 10A - 10B are, respectively, a back view and a cross-sectional side view of an exemplary article according to the embodiment shown in Figs. 9A-9B after heating to its transition temperature.
Fig. 11A - 11B are, respectively, a back view and a cross-sectional side view of an exemplary article according to one embodiment of the present disclosure before heating to its transition temperature.
Fig. 12A - 12B are, respectively, a back view and a cross-sectional side view of an exemplary article according to the embodiment shown in Figs. 11A-11B after heating to its transition temperature.

While the above-identified drawing figures set forth several embodiments of the present disclosure, other embodiments are also contemplated, as noted in the discussion. In all cases, this disclosure presents the disclosure by way of representation and not limitation. It should be understood that numerous other modifications and embodiments can be devised by those skilled in the art. The figures may not be drawn to scale. Like reference numbers may have been used throughout the figures to denote like parts.

### DETAILED DESCRIPTION

Nonwoven fiber webs according to the present disclosure may be of any type; for example, carded and cross-lapped fiber webs, melt blown webs, meltspun webs, air-laid fiber webs, wet-laid fiber webs, and combinations thereof. Methods for forming such nonwoven fiber webs are widely known and practiced in the art. The nonwoven web may have any thickness, density, or basis weight, but is typically in the form of a lofty open nonwoven fiber web. The nonwoven fiber web may include continuous and/or staple fibers. The nonwoven fiber web may comprise a single fiber type, or it may comprise a blend of two or more fiber types. For example, the nonwoven fiber web may be a blend of two or more fibers having different lengths and/or compositions. Individual fibers may include monocomponent fibers and polycomponent (typically bicomponent) fibers; for example, core-sheath fibers or splittable fibers. At least a portion of the fibers may be crimped. The nonwoven fiber web may be densified and otherwise strengthened; for example, by needletacking, stitchbonding, and/or a hot can or heated calender rolls which may bond the fibers together.

Examples of synthetic fiber materials include, polyesters (e.g., polyethylene terephthalate (PET)) and co-polyesters; polyolefins (e.g., polyethylene, polypropylene, poly(4-methyl-1-pentene)); polyamides (e.g., poly(hexamethylene adipamide), polycaprolactam, and aramids); acrylics (formed from a homopolymer or copolymer of acrylonitrile); rayon; polyvinylidene chloride-vinyl chloride copolymers; cellulosic esters (e.g., cellulose acetate); polycarbonates; polyurethanes; block copolymers such as, for example, styrene-butadiene-styrene and styrene-isoprene-styrene block copolymers; and combinations thereof. Examples of suitable natural fibers include cotton, wool, jute, coco, sisal, flax, and hemp. The fibers may include inorganic fibers such as glass fibers, carbon fibers, and ceramic fibers (e.g., as available under the trade designation NEXTEL from 3M Company of Saint Paul, MN). The fibers used may be virgin fibers or waste fibers reclaimed from garment cuttings, carpet manufacturing, fiber manufacturing, or textile processing, and so forth. The fineness or linear density of the fibers used may vary widely, depending upon the results desired.

Optionally the nonwoven fiber web may contain additional components such as, for example, fillers (e.g., activated charcoal or vermiculite), pigments, and fragrances.

In some embodiments, reinforcing strips are bonded to the nonwoven fiber web. Such reinforcing strips are typically flexible but substantially dimensionally stable at temperatures up to, and typically substantially above, Tₜᵣₐₙₛ. Examples of suitable materials for the reinforcing strips include metal (e.g., aluminum), plastics, wood, and combinations thereof.

Useful SMPs may be physically and/or chemically crosslinked. Suitable physically crosslinked SMPs include linear block copolymers such as thermoplastic polyurethane elastomers with hard segments and soft switching segments. Multiblock copolymers can also serve as SMPs such as, for example, polyurethanes with polystyrene and poly(1,4-butadiene) blocks; ABA triblock copolymers of poly(tetrahydrofuran) and poly(2- methyl-2-oxazoline); polyhedral oligomeric silsesquioxane (POSS)-modified polynorbornene; and polyethylene/Nylon-6 graft copolymers.

Other examples of shape-memory polymers include: polyurethanes, polynorbornenes, polyethers, polyacrylates, polyamides, polysiloxanes, polyether amides, polyether esters, trans-polyisoprenes, polymethyl methacrylates, cross-linked trans-polyoctylenes, cross-linked polyethylenes, cross-linked polycyclooctenes, inorganic-organic hybrid polymers, copolymer blends with polyethylene and styrene-butadiene co-polymers, urethane-butadiene co-polymers, polymethyl methacrylate, polycaprolactone, and oligocaprolactone copolymers.

Examples of suitable chemically crosslinked shape-memory polymers include, but are not limited to, crosslinked high density polyethylene, crosslinked low density polyethylene, and crosslinked copolymers of ethylene and vinyl acetate.

Suitable shape-memory polymers also include those described in U.S. Pat. Nos. 5,506,300 (Ward et al.); 5,145,935 (Hayashi); 5,665,822 (Bitler et al.); 6,160,084 (Langer); 6,388,043 (Langer); 5,155,199 (Hayashi); 7,173,096 (Mather et al.); 4,436,858 (Klosiewicz); 6,423,421 (Banaszak); and U.S. Pat. Appln. Publ. Nos. 2005/244353 (Lendlein et al.), U.S. 2007/009465 (Lendlein et al.), and 2006/041089 (Mather et al.).

Examples of commercially available thermoplastic SMPs include: polyurethanes available under the trade designation DIARY, including the MM, MP, MS, and MB (microbead powder) types series available from SMP Technologies, Inc. of Tokyo, Japan; elastic memory composites available under the trade designation EMC from Composite Technology Development, Inc. of Lafayette, CO; and polymers available under the trade designation VERIFLEX from Cornerstone Research Group, Inc. of Dayton, OH. The shape memory properties of acrylonitrile-butadiene-styrene (ABS) copolymers, polycarbonate, and polyethylene terephthalate are also disclosed by Hussein et al., in "New Technologies for Active Disassembly: Using the Shape Memory Effect in Engineering Polymers," Int. J. Product Development, 6:431-449 (2008).

Additional examples of commercially available shape memory polymer films that can be converted into various shapes such as, for example, strips and/or meshes include those heat shrink films available under the trade designations CORTUFF, CRYOVAC, and OPTI from Sealed Air Inc. of Elmwood Park, NJ.

The strip or strips, which may be interconnected or not, are bonded to the fiber web in a strained (deformed) temporary shape. This temporary shape is obtained by first forming the strip(s) in a desired intrinsic shape from a curable material or molten material and letting the material harden without applied stress. Next, the strip(s) are stressed (typically stretched in length and/or width) while heating at or above their Tₜᵣₐₙₛ, but below their Tᵤₚₚₑᵣ. Once strained to desired degree, the strained strip(s) are cooled to below Tₜᵣₐₙₛ (typically to ambient temperature). The strip(s) now have a temporary shape.

In some embodiments, films of shape memory polymer(s) can be stressed (typically stretched in length and/or width) while heating at or above their Tₜᵣₐₙₛ temperature, but below their Tᵤₚₚₑᵣ. Once strained to desired degree, the strained films are cooled to below Tₜᵣₐₙₛ (typically to ambient temperature) and cut into strips with the desired dimensions.

In some instances, films or strips of shape memory polymer(s) can be stressed (typically stretched in length and/or width) at a temperature below their Tₜᵣₐₙₛ temperature if sufficient force is applied.

The strips with the temporary shape are then bonded to the nonwoven fiber web. They may be bonded to the nonwoven fiber web by any suitable method, including: heat point bonding, ultrasonic point bonding, adhesive bonding using adhesive means such as, for example, thermosetting adhesive and/or glue; and/or mechanical fastening means such as, for example, stitching, staples, hooks, and/or rivets. The strip(s) may be bonded to the nonwoven fiber web continuously along its/their length or at discrete separated points.

At this point, the nonwoven fiber web is still essentially flat and can be stacked or rolled on a core, all convenient forms for storage, processing, and shipping. The nonwoven fiber web bonded to the strips may be converted at this stage or after thermal processing to restore the strip(s) to its/their intrinsic shape, which causes folding, cupping, and/or puckering of the nonwoven fiber web.

Thermal processing to restore the intrinsic shape (which may not be a complete restoration of the intrinsic shape) of the strips is accomplished by heating them to at least their Tₜᵣₐₙₛ temperature. Typically, the SMPs are chosen for specific applications such that their Tₜᵣₐₙₛ is below any temperature where significant degradation of the SMPs or articles including them occurs.

The choice of Tₜᵣₐₙₛ and Tᵤₚₚₑᵣ is not generally too important as long as Tᵤₚₚₑᵣ is greater than Tₜᵣₐₙₛ. Typically, Tₜᵣₐₙₛ is at least 40°C, and typically the difference between Tᵤₚₚₑᵣ and Tₜᵣₐₙₛ is at least about 20°C, however this is not a requirement.

Heating to restore the intrinsic shape of the strips may be accomplished by any suitable means such as, for example, heat gun, oven, induction heating, infrared heating, heated roller, microwave heating, heated platen, steam, and combinations thereof.

The disclosure will now be further illustrated by way of the Figures.

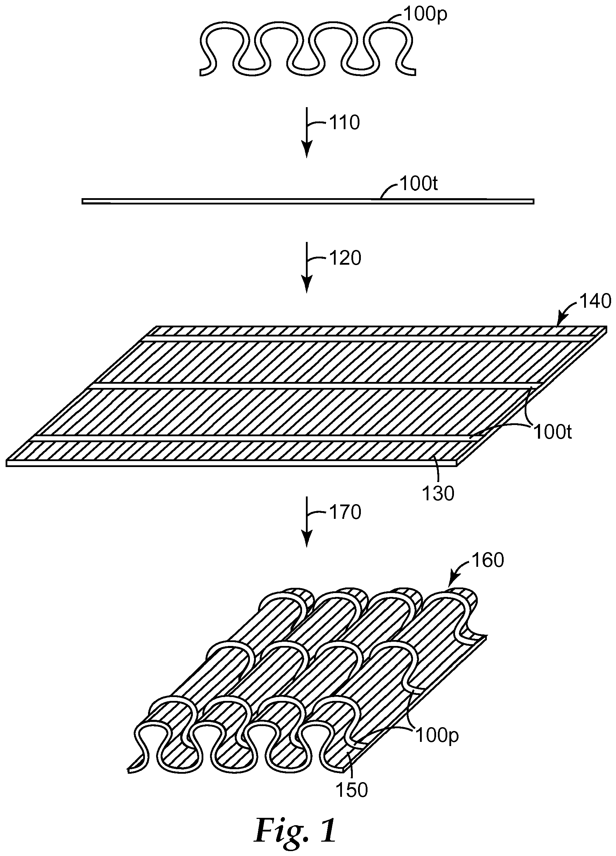

Fig. 1 is a process flow diagram showing an exemplary method of making an article according to the present disclosure. A strip lOOp comprising a shape-memory polymer, formed in its intrinsic shape, is stressed 110 at a temperature above its Tₜᵣₐₙₛ but below its Tᵤₚₚₑᵣ and cooled such that it forms an elongated strip 100t. Strips lOOt are bonded 120 (e.g., by lamination) to nonwoven fiber web 130 in a substantially parallel arrangement to form a composite web 140. The composite web 140 is then heated 170 above Tₜᵣₐₙₛ and the strips lOOt revert at least partially to their intrinsic shape, thereby forming folds in web 150 and providing a pleated fiber web 160 that is suitable for use, for example in a filter.

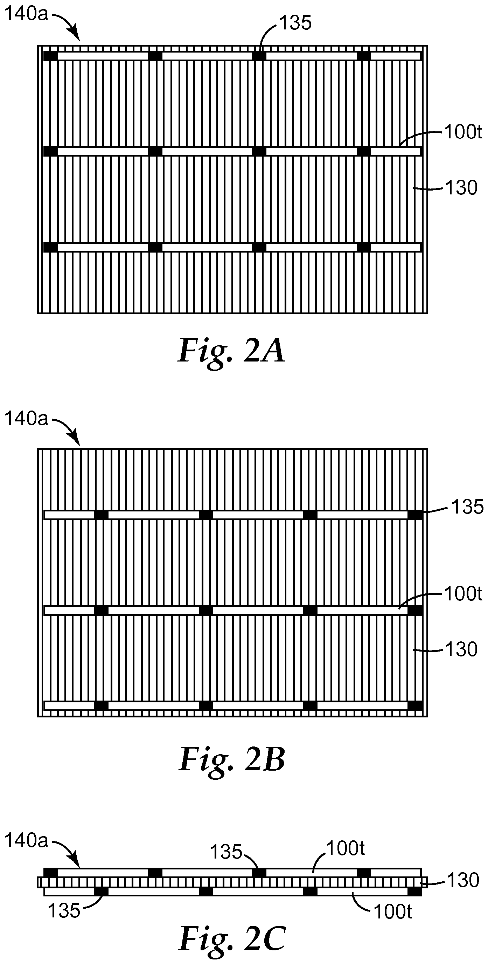

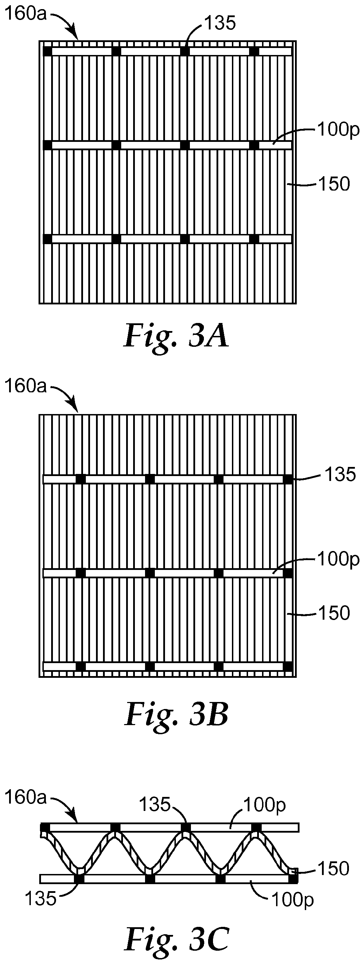

Figs. 2A-2C show one exemplary composite web 140a. Strips lOOt are bonded to nonwoven fiber web 130 at bonded points 135. Fig. 2A is a top view, Fig. 2B is a bottom view, and Fig. 2C is a side view of composite web 140a. When composite web 140a is heated above Tₜᵣₐₙₛ, strips lOOt shrink and revert to strips lOOp, which is shown in Figs. 3A-3C. Fig. 3A is a schematic top view, Fig. 3B is a schematic bottom view, and Fig. 3C is a schematic side view of resultant pleated fiber web 160a.

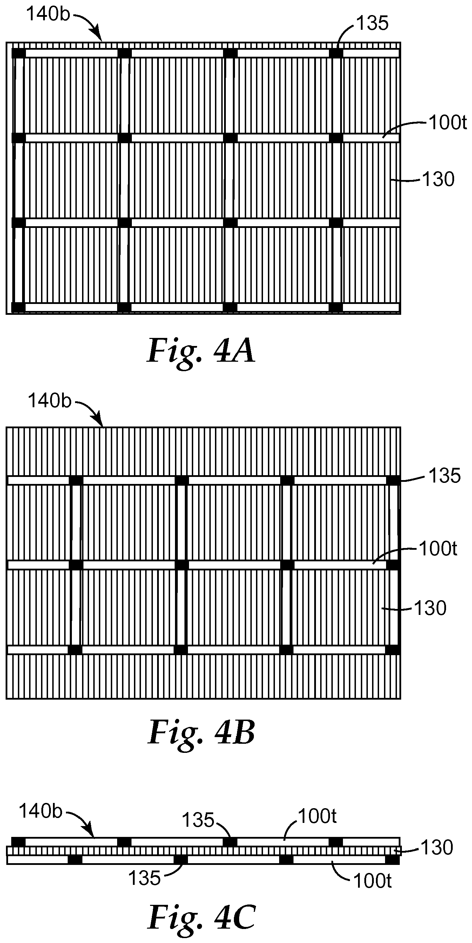

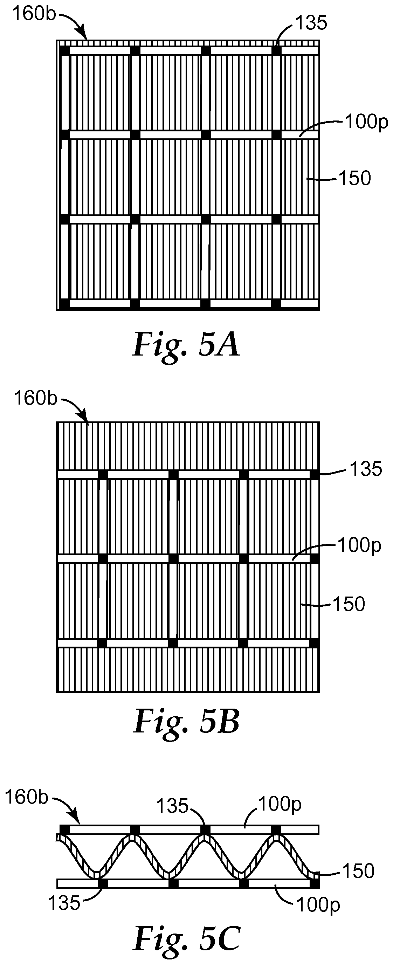

Figs. 4A - 4C show another exemplary embodiment of composite web 140, Fig. 4A is a schematic top view, Fig. 4B is a schematic bottom view, and Fig. 4C is a schematic side view of composite web 140b. When composite web 140b is heated above Tₜᵣₐₙₛ, strips lOOt shrink and revert to strips lOOp, as shown in Figs. 5A-5C. Fig. 5A is a schematic top view, Fig. 5B is a schematic bottom view, and Fig. 5C is a schematic side view of cupped and/or puckered fiber web 160b.

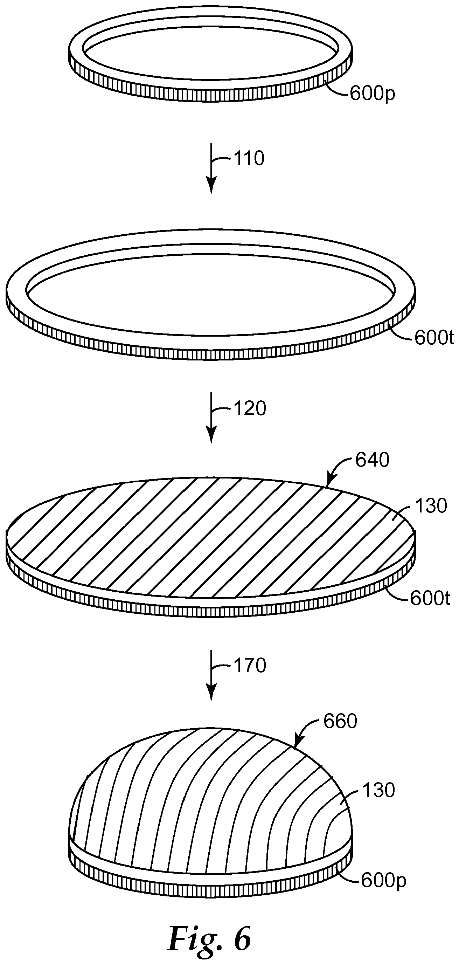

Fig. 6 is a process flow diagram showing another exemplary method of making an article according to the present disclosure. A substantially circular strip 600p comprising a shape-memory polymer, formed in its intrinsic shape, is outwardly stressed 110 at a temperature above its Tₜᵣₐₙₛ, but below its Tᵤₚₚₑᵣ, and cooled such that its size is enlarged forming strip 600t. Strip 600t is bonded 120 (e.g., by lamination) to nonwoven fiber web 130 to form a composite web 640. The composite web 640 is then heated 170 above Tₜᵣₐₙₛ and the strip 600t reverts at least partially to its intrinsic shape, thereby forming a cupped and/or puckered fiber web 660 that is suitable for use, for example, in making a respirator mask.

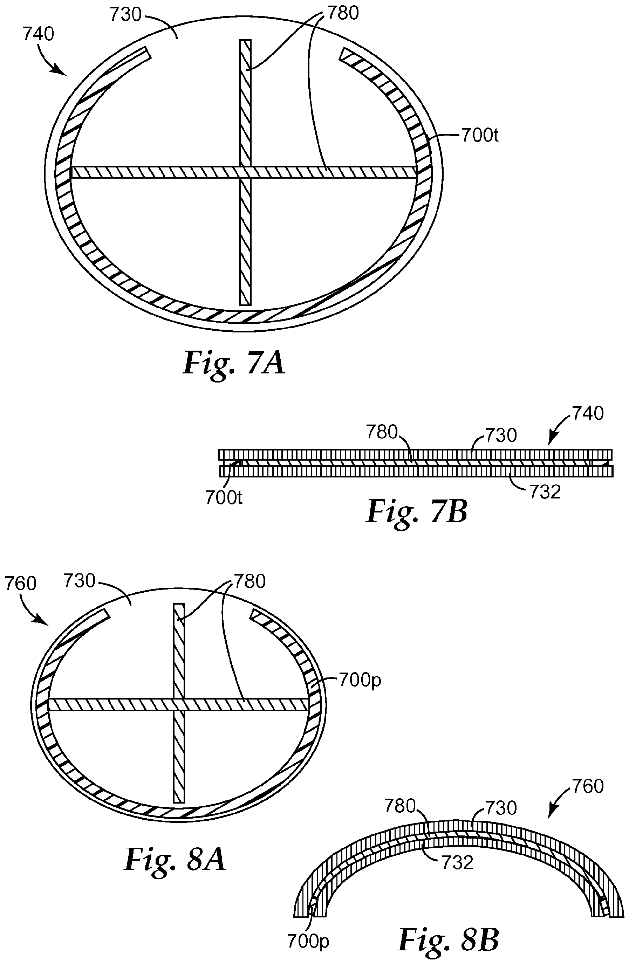

Figs. 7A - 7B show another exemplary embodiment of composite web 740, Fig. 7A is a cross-sectional back view and Fig. 7B is a cross-sectional side view of composite web 740. In this embodiment, reinforcing strips 780 and strip 700t are bonded between nonwoven fiber webs 730, 732. When composite web 740 is heated above Tₜᵣₐₙₛ, strip 700t shrinks and reverts to strip 700p as shown in Figs. 8A-8B thereby forming a pucker in nonwoven fiber webs 730, 732 and providing cupped and/or puckered fiber web 760 that is suitable for use, for example, in making a respirator mask. Fig. 8A is a cut-away back view and Fig. 8B is a cross-sectional side view of puckered fiber web 760.

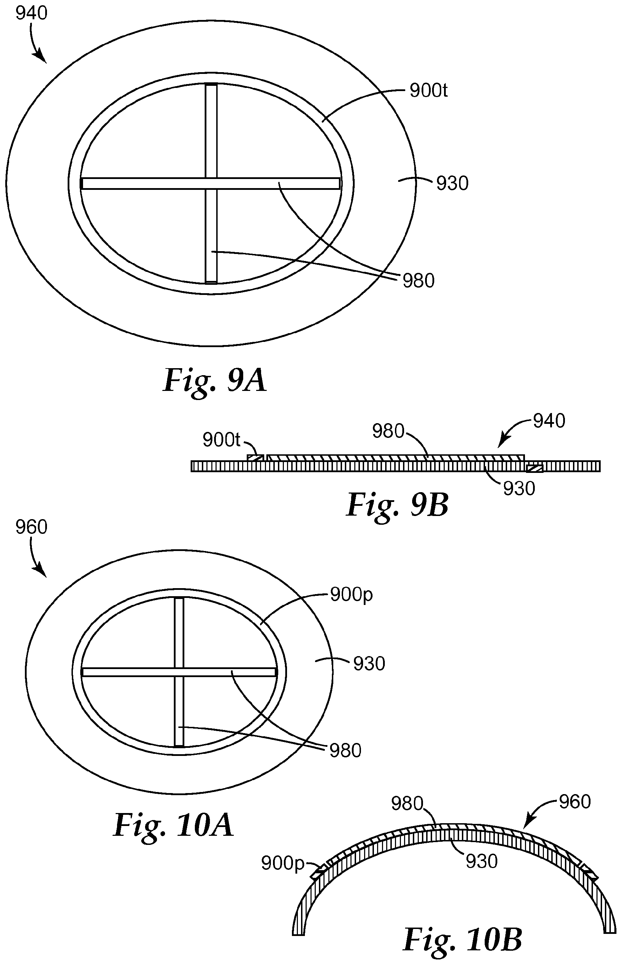

Figs. 9A - 9B show another exemplary embodiment of composite web 940, Fig. 9A is a back view and Fig. 9B is a cross-sectional side view of composite web 940. In this embodiment, reinforcing strips 980 and temporary shaped strip 900t are bonded to nonwoven fiber web 930. When composite web 940 is heated above Tₜᵣₐₙₛ, strip 900t shrinks in thickness and expands in length and width, reverting to intrinsically shaped strip 900p as shown in Figs. 10A-10B. This forms cupped and/or puckered fiber web 960 that is suitable for use, for example, in making a respirator mask. Fig. 10A is a back view and Fig. 10B is a cross-sectional side view of fiber web 960.

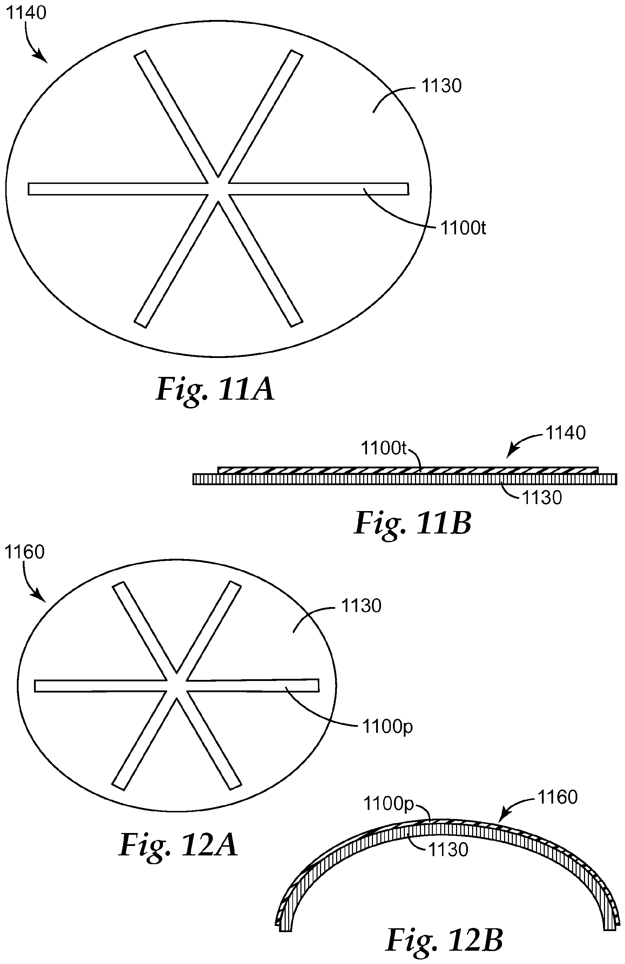

Figs. 11A - 11B show another exemplary embodiment of composite web 1140, Fig. 11A is a back view and Fig. 11B is a cross-sectional side view of composite web 1140. In this embodiment, strained temporary strips 1100t are bonded to nonwoven fiber web 1130. When composite web 1140 is heated above Tₜᵣₐₙₛ, temporary strips 1100t shrink in width and elongate in length, reverting to intrinsically shaped strips 1100p as shown in Figs. 12A-12B thereby forming a cup and/or pucker in nonwoven fiber web 1130 and providing cupped and/or puckered fiber web 1160 that is suitable for use, for example, in making a respirator mask. Fig. 12A is a back view and Fig. 12B is a cross-sectional side view of fiber web 1160.

Objects and advantages of this disclosure are further illustrated by the following non-limiting examples.

### EXAMPLES

Unless otherwise noted, all parts, percentages, ratios, etc. in the Examples and the rest of the specification are by weight. Solvents and other reagents used were obtained from Sigma-Aldrich Chemical Company; Milwaukee, WI unless otherwise noted.

### EXAMPLE 1

CORTUFF heat shrink film with 0.003 inch (0.08 mm) thickness from Sealed Air Corp. of Elmwood, NJ, was cut into strips that were 0.5 inch (13 mm) wide and 12 inches (300 mm) long. One of these strips was positioned on the longer centerline of one face of a 10 inches (250 mm) by 12 inches (300 mm) piece of nonwoven filter media obtained by removal of the metal mesh from a 3M FILTRETE 9800 filter from 3M Company of Saint Paul, MN, and an additional two strips were positioned parallel to the first strip and 3 inches (76 mm) away from either side of the first strip. The three strips were attached to the media with staples at the end of each strip and on 2-inch (50-mm) intervals across the length of the strips. An additional four strips were then positioned on the opposite face of the filter media, and parallel to the first strips. Two of these strips were positioned 1.5 inches (38 mm) from each side of the centerline, and the other two strips were positioned 0.5 inch (13 mm) from each longer edge of the media. These strips were attached to the media with staples positioned one inch from each end of the strips and, from that point, on 2-inch (50-mm) intervals across the length of the strips. The assembly was then put into a 140°C oven for two minutes (min), during which time the strips shortened and caused the media to pleat.

### EXAMPLE 2

CORTUFF heat shrink film with 0.003 inch (0.08 mm) thickness from Sealed Air Corp. was placed in a 125°C oven for 15 minutes, during which time it shrank to 30 percent of its original length and width. A KARO IV Laboratory Stretching Machine from Bruckner Inc. of Greenville, SC, was then used to stretch the film at 120°C to twice its original length while holding the width constant. This oriented film was cut into two 9 inches x 9 inches (230 mm x 230 mm) pieces. The films were die cut with 0.5 inch x 2.5 inches (13 mm x 64 mm) rectangular openings with 0.25 inch (6 mm) of material left between each opening. The openings were positioned in 3 x 14 array, and the longer dimension of the rectangular openings was parallel to the direction in which the film had been stretched. One of the resulting films was positioned over a 9 inches x 10.5 inches (23 cm x 26.7 cm) piece of nonwoven filter media obtained by removal of the metal mesh from a 3M FILTRETE 9800 filter with the longer dimension of the rectangular openings aligned with the longer dimension of the media. The film and the media were aligned on three of the four edges. The film was stapled to the nonwoven filter media at 2.75-inch (69.9-mm) intervals such that the staples passed through the film only at the ends of the rectangular openings. A second piece of the same film was positioned on the opposite face of the filter media and aligned on three of the four edges including the edge that was not affixed to the first film. It was similarly attached with staples only at the ends of the rectangular openings. The resulting flat assembly was placed in a 130°C oven for 10 min during which time the films shrank to 4.5-5.5 inches x 8.0 inches-8.5 inches (11-14 cm x 20 cm x 22 cm) and the filter media became pleated.

### EXAMPLE 3

A monomer solution was prepared from IRGANOX 1010 antioxidant (1.6 grams (g), available from Ciba Specialty Chemicals Corp. of Tarrytown, NY) in cyclooctene (40 milliliters (mL)) and dicyclopentadiene (120 mL). To this was added a solution of (1,3-bis(2,4,6-trimethylphenyl)-2-imidazolidinylidene)dichloro(phenylmethylene)-(tricyclohexylphosphine)ruthenium (Grubbs Second Generation Catalyst, 96 mg) in toluene (1.6 mL). The catalyzed solution was immediately transferred to a mold composed of two aluminum plates separated by a 0.015 inch (0.38 mm) thick spacer with a 6 inches × 11 inches (150 mm x 280 mm) rectangular opening. After one hour, the mold was placed in a 60°C oven for 30 min. The resulting polymer film was removed from the mold and returned to the oven for an additional 15 min. The film was then stretched at 80°C to 1.5 times its original length and width using a KARO IV laboratory stretching machine available from Bruckner Inc.

This stretched film was cut into two curved strips 0.25 inch (6 mm) wide such that the strips could be positioned to form an arc of 310° that followed an ellipse with major and minor diameters of 6.8 inches (170 mm) and 5.4 inches (140 mm). The portion of the ellipse that was not covered by the arc was centered on the minor diameter. These strips were then adhered to a piece of nonwoven filter media in the shape of an ellipse with major and minor diameters of 7.8 inches (200 mm) and 6.4 inches (160 mm). The nonwoven filter media was a three-layer laminate construction, with a top layer consisting of a 50 grams per square meter (gsm) polypropylene spunbond coverweb available from BBA Nonwovens Simpsonville, Inc. of Simpsonville, SC; a middle layer consisting of a blown microfiber web made from polypropylene TOTAL 3960 from Total Petrochemicals of Houston, TX, that had been corona treated and hydrocharged as described in U.S. Pat. No. 5,496,507 (Angadjivand et al.), except that the effective fiber diameter (efd) was 8 microns, and the basis weight was 50 gsm); and a bottom layer consisting of 17 gsm polypropylene spunbond coverweb from BBA Nonwovens. Two strips of polypropylene from Plastics International of Eden Prairie, MN, 0.03 inch x 0.2 inch x 4 inches (0.8 mm x 5 mm x 100 mm) were adhered to the center of the opposite face of the filter media in a cross shape. The flat assembly was then placed in an 80°C oven for 5 min, during which time the polymer arc contracted and forced the media into a dome shape.

### EXAMPLE 4

The top layer was removed from a piece of the nonwoven filter media (three-layer laminate construction) used in Example 3 having an elliptical shape of major and minor diameters of 8.5 inches (22 cm) and 7.0 inches (18 cm). Polypropylene strips from Plastics International, 0.03 inch x 0.25 inch x 6.3 inches (0.76 mm x 6.4 mm x 16 cm) and 0.03 inch x 0.25 inch x 5.2 inches (0.76 mm x 6.4 mm x 13 cm) were placed in a cross shape on the center of the exposed middle layer. CORTUFF brand heat shrink film with 0.003 inch (0.08 mm) thickness was cut into a 0.25 inch (6.4 mm) wide strip in the shape of an arc of 310° that followed an ellipse with major and minor diameters of 7.5 inches (19 cm) and 6.0 inches (15 cm). The portion of the ellipse that was not covered by the arc was centered on the minor diameter. This strip was placed on the middle layer generally as shown in Fig. 7A. Adhesive available as SCOTCH MAXIMUM STRENGTH ADHESIVE 6047, from 3M Company was applied to the CORTUFF brand heat shrink film strip, polypropylene strips and media, and the top layer of the filter media was then replaced over this assembly. After one hour, the resulting flat assembly was heated in a 110°C oven for 2 min, during which time the CORTUFF heat shrink film strips contracted and forced the media in to a dome shape.

### EXAMPLE 5

A mold was made from a 1/2 inch (13 mm) thick aluminum plate by milling a 1/8 inch (3.2 mm) wide channel with a period of 1.06 inches (2.69 cm) all the way through the aluminum plate with a shape generally shown in lOOp of Fig. 1. One face of the milled plate was bolted to a 1/8 inch (3.2 mm) thick aluminum plate to form an open-faced mold.

A monomer solution was prepared from IRGANOX 1010 antioxidant (1.7 g) in cyclooctene (12.8 mL) and dicyclopentadiene (44.2 mL). To this monomer solution was added a solution of 10 percent tricyclohexylphosphine in toluene (0.7 mL). The resultant solution was cooled in an ice bath and degassed under vacuum. Then a solution of (1,3-bis(2,4,6-trimethylphenyl)-2-imidazolidinylidene)dichloro(phenylmethylene)-(tricyclohexylphosphine)ruthenium (Grubbs Second Generation Catalyst, 30 mg) in toluene (0.6 mL) was added, and the catalyzed solution was immediately transferred to the mold. Once the solution gelled, the mold was placed in a 110 °C oven to post cure for 10 min. A shaped polymer resulted, which was then forced out of the mold.

The shaped polymer was pressed in a Carver press from Carver, Inc. of Wabash, IN, at 100°F (38°C) for 10 min to give a flat 1/8 inch × 1/2 inch × 24 inches (3.2 mm x 13 mm x 61 cm) strip. The strip was then cut to give four strips of 1/8 inch × 1/4 inch × 12 inches (3.2 mm x 6.4 mm x 30 cm). These were glued to a 12 inches × 11 inches (30 cm x 28 cm) piece of nonwoven material (same as used in Example 1) using SCOTCH MAXIMUM STRENGTH ADHESIVE 6047 and allowed to cure overnight. The resulting flat assembly was then heated in a 65°C oven for 15 min, and it became a pleated filter. It was post-cured in a 110°C oven for 15 min more, and the pleats became nearly as narrow as the pattern in the original mold.

### EXAMPLE 5

A mold was made from a 1/2 inch (13 mm) thick aluminum plate by milling a 1/8 inch (3.2 mm) wide channel with a period of 1.06 inches (2.69 cm) all the way through the aluminum plate with a shape generally shown in lOOp of Fig. 1. One face of the milled plate was bolted to a 1/8 inch (3.2 mm) thick aluminum plate to form an open-faced mold.

A monomer solution was prepared from IRGANOX 1010 antioxidant (1.7 g) in cyclooctene (12.8 mL) and dicyclopentadiene (44.2 mL). To this monomer solution was added a solution of 10 percent tricyclohexylphosphine in toluene (0.7 mL). The resultant solution was cooled in an ice bath and degassed under vacuum. Then a solution of (1,3-bis(2,4,6-trimethylphenyl)-2-imidazolidinylidene)dichloro(phenylmethylene)-(tricyclohexylphosphine)ruthenium (Grubbs Second Generation Catalyst, 30 mg) in toluene (0.6 mL) was added, and the catalyzed solution was immediately transferred to the mold. Once the solution gelled, the mold was placed in a 110 °C oven to post cure for 10 min. A shaped polymer resulted, which was then forced out of the mold.

The shaped polymer was pressed in a Carver press from Carver, Inc. of Wabash, IN, at 100°F (38°C) for 10 min to give a flat 1/8 inch × 1/2 inch × 24 inches (3.2 mm x 13 mm x 61 cm) strip. The strip was then cut to give four strips of 1/8 inch × 1/4 inch × 12 inches (3.2 mm x 6.4 mm x 30 cm). These were glued to a 12 inches × 11 inches (30 cm x 28 cm) piece of nonwoven material (same as used in Example 1) using SCOTCH MAXIMUM STRENGTH ADHESIVE 6047 and allowed to cure overnight. The resulting flat assembly was then heated in a 65°C oven for 15 min, and it became a pleated filter. It was post-cured in a 110°C oven for 15 min more, and the pleats became nearly as narrow as the pattern in the original mold.

## Claims

1. An article comprising:
a nonwoven fiber web; and
strips bonded to the nonwoven fiber web, wherein the strips comprise a shape-memory polymer, wherein each strip respectively has a strained temporary shape and an intrinsic shape, and wherein if heated at or above a transition temperature, the strips at least partially convert from their respective strained temporary shape to their respective intrinsic shape, thereby causing at least one of folding or puckering of the nonwoven fiber web, wherein at least a portion of the strips are interconnected.

2. The article of claim 1, wherein the strips are linear.

3. The article of claim 1, wherein the strips are disposed entirely on one major surface of the nonwoven fiber web.

4. A method of making an article comprising bonding strips to a nonwoven fiber web, wherein the strips comprise a shape-memory polymer, wherein each strip respectively has a strained temporary shape and an intrinsic shape, and wherein if heated at or above a transition temperature, the strips at least partially convert from their respective strained temporary shape to their respective intrinsic shape, thereby causing at least one of folding, cupping, or puckering of the nonwoven fiber web, wherein at least some of the strips are interconnected.

5. The method of claim 4, wherein the strips comprise a mesh.

## Patentansprüche

1. Artikel, umfassend:
eine Faservliesbahn; und
mit der Faservliesbahn verklebte Streifen, wobei die Streifen ein Formgedächtnis-Polymer umfassen, wobei jeder Streifen eine vorübergehend gespannte Form und eine natürliche Form aufweist, und wobei die Streifen beim Erwärmen auf oder über eine Übergangstemperatur mindestens teilweise von ihrer jeweiligen vorübergehend gespannten Form in ihre jeweilige natürliche Form übergehen und dadurch mindestens eines von Falten oder Kräuseln der Faservliesbahn bewirken, wobei mindestens ein Teil der Streifen untereinander verbunden ist.

2. Artikel nach Anspruch 1, wobei die Streifen linear sind.

3. Artikel nach Anspruch 1, wobei die Streifen vollständig auf einer Hauptoberfläche der Faservliesbahn angeordnet sind.

4. Verfahren zum Herstellen eines Artikels, umfassend das Kleben von Streifen auf eine Faservliesbahn, wobei die Streifen ein Formgedächtnis-Polymer umfassen, wobei jeder Streifen eine vorübergehend gespannte Form und eine natürliche Form aufweist, und wobei die Streifen beim Erwärmen auf oder über eine Übergangstemperatur mindestens teilweise von ihrer jeweiligen vorübergehend gespannten Form in ihre jeweilige natürliche Form übergehen, und dadurch mindestens eines von Falten, Wellen oder Kräuseln der Faservliesbahn bewirken, wobei mindestens einige der Streifen untereinander verbunden sind.

5. Verfahren nach Anspruch 4, wobei die Streifen ein Maschengewebe umfassen.

## Revendications

1. Article comprenant :
une toile de fibres non tissées ; et
des bandes liées à la toile de fibres non tissées, dans lequel les bandes comprennent un polymère à mémoire de forme, dans lequel chaque bande a respectivement une forme temporaire contrainte et une forme intrinsèque, et dans lequel si elles sont chauffées au niveau ou au-dessus d'une température de transition, les bandes passent au moins partiellement de leur forme temporaire contrainte respective à leur forme intrinsèque respective, en provoquant de ce fait au moins l'un parmi un pliage ou un plissement de la toile de fibres non tissées, dans lequel au moins une partie des bandes sont interconnectées.

2. Article selon la revendication 1, dans lequel les bandes sont linéaires.

3. Article selon la revendication 1, dans lequel les bandes sont disposées entièrement sur une surface principale de la toile de fibres non tissées.

4. Procédé de fabrication d'un article comprenant la liaison de bandes sur une toile de fibres non tissées, dans lequel les bandes comprennent un polymère à mémoire de forme, dans lequel chaque bande a respectivement une forme temporaire contrainte et une forme intrinsèque, et dans lequel, si elles sont chauffées au niveau ou au-dessus d'une température de transition, les bandes passent au moins partiellement de leur forme temporaire contrainte respective à leur forme intrinsèque respective, en provoquant de ce fait au moins l'un parmi un pliage, un bombement ou un plissement de la toile de fibres non tissées, dans lequel au moins certaines des bandes sont interconnectées.

5. Procédé selon la revendication 4, dans lequel les bandes comprennent un maillage.
